# EUROPÄISCHE PATENTANMELDUNG

(11) **EP 1 712 910 A1**
(43) Veröffentlichungstag der Anmeldung: **18.10.2006**
(21) Anmeldenummer: 05007962.3
(22) Anmeldetag: 12.04.2005
(51) Int. Cl.: G01N 33/487, G01N 27/327

(54) **Analysegerät zur Analyse einer Probenflüssigkeit mittels eines Testelements**

(71) Anmelder: F.HOFFMANN-LA ROCHE AG, 4070 Basel (CH); Roche Diagnostics GmbH, 68305 Mannheim (DE)
(72) Erfinder: Jansen, Paul, 68163 Mannheim (DE)
(74) Vertreter: Isenbruck, Günter

(57) **Zusammenfassung**

Die Erfindung bezieht sich auf ein Analysegerät zur Analyse einer Probenflüssigkeit (8) mittels eines Testelements (5). Das Analysegerät umfasst eine Messanordnung zur Durchführung von Messungen an einer auf ein Testelement (5) aufgenommenen Probenflüssigkeit (8). Die Messanordnung ist in einem Gehäuse (1) aufgenommen und umfasst eine Testelementaufnahmeeinheit. In der Testelementaufnahmeeinheit kann das Testelement zumindest eine Probenaufgabeposition und eine Analyseposition einnehmen. Ferner weist das Analysegerät ein beweglich mit dem Gehäuse (1) verbundenes Abfallbehältnis (12) zum Aufnehmen von Testelementen (5, 17) nach der Durchführung von Messungen an der Probenflüssigkeit (8) auf den Testelementen (5) in der Analyseposition auf. Das Abfallbehältnis (12) ist so angeordnet, dass ein Probenaufnahmeort eines in der Probenaufgabeposition positionierten Testelements (5) zugänglich ist.

## Beschreibung

Die Erfindung bezieht sich auf ein Analysegerät zur Analyse einer Probenflüssigkeit mit einem Abfallbehältnis zur Aufnahme von verbrauchten Testelementen.

Zur Analyse von Proben, beispielsweise Körperflüssigkeiten wie Blut oder Urin, werden häufig Testelement-Analysegeräte verwendet, bei denen die zu analysierenden Proben auf ein Testelement gegeben werden und gegebenenfalls mit einer oder mehreren Reagenzien in einem Testfeld auf dem Testelement reagieren, bevor sie analysiert werden. Die optische, insbesondere photometrische, und die elektrochemische Auswertung von Testelementen stellen die gebräuchlichsten Verfahren zur schnellen Bestimmung der Konzentration von Analyten in Proben dar. Photometrische und elektrochemische Auswertungen werden allgemein im Bereich der Analytik, der Umweltanalytik und vor allem im Bereich der medizinischen Diagnostik eingesetzt. Insbesondere im Bereich der Blutglukösediagnostik aus Kapillarblut besitzen Testelemente, die photometrisch oder elektrochemisch ausgewertet werden, einen großen Stellenwert.

In den letzten Jahren haben tragbare Messgeräte zur Blutzuckerbestimmung an Bedeutung gewonnen. Sie ermöglichen zu jeder Zeit mittels eines einfach zu bedienenden Messgerätes, einer hinsichtlich des Einstechschmerzes optimierten Stechhilfe und eines Testelements für den einmaligen Gebrauch, Blutzuckermesswerte zu bestimmen und damit eine genauere Insulindosierung des Patienten zur Stabilisierung seines Blutzuckerwertes vorzunehmen. Die Mehrzahl derzeit gebräuchlicher Blutzuckermessgeräte sieht getrennte Einzel-Testelemente, Messgeräte und Stechhilfen vor. Die Einzel-Testelemente werden hierbei von dem Patienten aus einer feuchtigkeitsgeschützten Einzelverpackung entnommen. Blut wird durch einen Einstich mit einer Stechhilfe gewonnen. Hierauf wird eine erforderliche Mindestmenge an Blut auf das Testelement aufgebracht und eine Messung mit Hilfe des Messgerätes durchgeführt.

Es gibt verschiedene Formen von Testelementen. Bekannt sind z.B. im Wesentlichen quadratische Blättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Zur räumlichen Trennung von Detektionszone und Probenaufgabestelle eines Testelements sind im Stand der Technik kapillare Testelemente bekannt, z.B. aus WO 99/29429.

Die Verpackung des jeweiligen Testelements ist so konzipiert, dass sie die wesentlichen Aufgaben zum Erhalt der Funktion der chemischen und biochemischen Bestandteile auf dem Testelement während einer längeren Lagerzeit erfüllt. Diese Aufgaben sind vor allem Schutz vor der Einwirkung von Lichtstrahlen, Schutz vor dem Zutritt von Feuchtigkeit, Schmutz, Keimen und Staub, sowie Schutz vor mechanischer Beeinträchtigung der Testelemente.

Alternativ zu Einzelverpackungen sind Vorratsbehälter bekannt, welche eine Vielzahl von einzeln entnehmbaren Testelementen beinhalten und einen ausreichend großen Trockenmittelvorrat vorsehen, um die durch Öffnen und Entnahme eines Testelements eingeführte Feuchtigkeit zu absorbieren und somit eine ausreichende Lagerzeit für alle im Behälter vorgesehenen Testelemente zu gewährleisten. Ein solcher Vorratsbehälter ist aus EP 0 640 393 B1 bekannt. In dem Vorratsbehältnis stecken die Testelemente wie in einem Köcher, aus dem sie bei geöffnetem Bevorratungssystem entnommen werden können.

Diese separaten Vorratsbehälter haben den Nachteil, dass die einzelnen Testelemente umständlich manuell entnommen werden müssen. Ein Patient, der z.B. einen Blutzuckertest durchführen möchte, muss neben dem Messgerät eine Stechhilfe und einen getrennten Testelement-Vorratsbehälter mit sich führen. Neben dieser Unbequemlichkeit ist es vor allem nachteilig, dass bei Entnahme eines Testelements dieses und/oder ein anderes verunreinigt werden und die Verunreinigung zu falschen Messresultaten führen kann. Es besteht die Gefahr der Kontamination von Teststreifen durch an den Händen des Patienten haftendem Schmutz und aufgrund des Herausfallens des Testelements.

Als Alternative ist die Lagerung einer bestimmten Anzahl an Testelementen im Messgerät selbst bekannt.

DE 198 19 407 offenbart einen Behälter für Blutzuckermessgeräte oder andere Messgeräte, welche mit Einweg-Teststreifen arbeiten, die zur Messung einem Sensor zuführbar sind, wobei der Behälter aus zwei Teilen besteht, in dessen erstem die Teststreifen magaziniert sind und in dessen zweitem die verbrauchten Teststreifen gesammelt werden. Dabei können die Teststreifen so aneinandergereiht sein, dass sie ein Band bilden, welches ähnlich dem Band in einer Tonbandkassette gespult werden kann. Sie können stattdessen auch so angeordnet sein, dass sie eine runde Scheibe bilden, auf der sie in einem definierten Abstand zueinander im Bereich des Scheibenumfangs angeordnet sind, so dass durch Drehen der Scheibe ein neues Testfeld in die entsprechende Messposition kommt. Eine weitere Möglichkeit ist, dass die Teststreifen einen Stapel bilden, welcher durch eine Mechanik einzeln abgearbeitet wird und die Teststreifen nacheinander in die entsprechende Messposition und nach Erfolgen der Messung in ein Sammelfach bringt.

In DE 198 54 316 A1 wird ein Vorratsbehältnis mit separaten wasserdampfdichten Kammern für Testelemente beschrieben. Jede der Kammern weist zumindest zwei gegenüberliegende, durch jeweils eine Siegelfolie verschlossene Öffnungen auf. Zur Entnahme der Testelemente erfolgt ein Herausschieben eines Testelements aus seiner Kammer mit Hilfe eines Stößels. Der Stößel durchtrennt dabei die Siegelfolie auf der einen Seite der Kammer und drückt dann auf das Testelement, das aufgrund dieses Drucks des Stößels die Siegelfolie auf der gegenüberliegenden Seite durchtrennt, so dass das Testelement aus der Kammer herausgeschoben werden kann. Weitere Mechanismen zur automatischen Testelemententnahme aus einem Vorratsbehältnis offenbaren EP 0 738 666 B1, DE 197 15 031 A1, US 5,575,403, DE 199 02 601 A1 und DE 43 26 339 A1.

Nach der Aufnahme einer Probe (z.B. Blut) auf das entnommene Testelement erfolgt die Detektion und Messdatenauswertung in dem Analysegerät. Die verbrauchten Testelemente werden im Stand der Technik üblicherweise aus dem Analysegerät ausgeworfen oder manuell entnommen und müssen einzeln entsorgt werden. Es fällt somit nach jeder Messung ein mit Blut, Kontrolllösung oder einer sonstigen Probenflüssigkeit kontaminiertes Testelement an. Dies kann für einen Anwender unangenehm sein, der das Analysegerät in der Öffentlichkeit verwenden muss und der aus Hygiene- und Diskretionsgründen solche verbrauchten Testelemente nicht öffentlich handhaben möchte.

Aufgabe der vorliegenden Erfindung ist es, ein Analysegerät bereitzustellen, das die Nachteile des Standes der Technik vermeidet und das insbesondere eine hygienische und diskrete Entsorgung von verbrauchten Testelementen erlaubt.

Diese Aufgabe wird erfindungsgemäß gelöst durch ein Analysegerät zur Analyse einer Probenflüssigkeit mittels eines Testelements, umfassend eine Messanordnung zur Durchführung von Messungen an einer auf einem Testelement aufgenommenen Probenflüssigkeit, wobei die Messanordnung in einem Gehäuse aufgenommen ist und eine Testelementaufnahmeeinheit umfasst, in der das Testelement zumindest eine Probenaufgabeposition und eine Analyseposition einnehmen kann.

Ferner umfasst das Analysegerät ein beweglich mit dem Gehäuse verbundenes Abfallbehältnis zum Aufnehmen von Testelementen nach der Durchführung von Messungen an der Probenflüssigkeit auf den Testelementen in der Analyseposition, wobei das Abfallbehältnis so angeordnet ist, dass ein Probenaufnahmeort eines in der Probenaufgabeposition positionierten Testelements zugänglich ist.

In dem erfindungsgemäßen Analysegerät können beliebige, dem Fachmann bekannte Testelemente verwendet werden. Vorzugsweise handelt es sich um einmal verwendbare Teststreifen mit einem eine Testchemie enthaltenden Testfeld, die zur photometrischen oder elektrochemischen Analyse einer Probenflüssigkeit auf dem Testfeld vorgesehen sind.

Das in dem erfindungsgemäßen Analysegerät ggf. enthaltene Vorratsbehältnis ist beispielsweise ein trommelförmiges oder stapelförmiges Magazin, in dem mindestens 2, bevorzugt zwischen 10 und 100 Testelemente aufbewahrt werden können. Die Testelemente können vorzugsweise zur Verwendung automatisch aus dem Vorratsbehältnis entnommen werden, z.B. durch einen Stößel oder Schieber in dem Analysegerät. Das erfindungsgemäße Analysegerät kann jedoch auch kein Vorratsbehältnis enthalten, wobei ein Benutzer unverbrauchte Testelemente in einem gesonderten Vorratsbehältnis mit sich führen und zur Messung manuell in die Testelementaufnahmeeinheit einlegen muss.

In der Regel enthalten die Testelemente Reagenzien, deren Reaktion mit der Probenflüssigkeit zu einer physikalisch nachweisbaren Veränderung des Analyten führt, die mit Hilfe der Messanordnung gemessen wird. Die Messanordnung ist z.B. eine Messanordnung zur photometrischen oder elektrochemischen Analyse einer auf dem Testelement vorhandenen Probe. Bei photometrischen Analysesystemen wird z.B. eine Farbänderung photometrisch gemessen, die durch eine Reaktion des Analyten in dem Testfeld des Testelements verursacht wird. Die dazu verwendete photometrische Messanordnung umfasst üblicherweise eine Lichtquelle, optische Elemente und einen Detektor zur Detektion von an dem Testfeld reflektiertem oder durch das Testfeld transmittiertem Licht. Bei elektrochemischen Analysesystemen findet in Folge der Reaktion eine als Spannung oder Stromstärke messbare elektrochemische Veränderung des Analyten in dem Testfeld statt. Die Messanordnung wirkt dabei mit auf dem Testelement vorhandenen Leitungselementen, beispielsweise gedruckten oder geätzten Leiterbahnen zusammen, die mit der Messanordnung verbunden werden. Das gemessene elektrische Signal wird dort gegebenenfalls verstärkt, analysiert und angezeigt.

Die Messanordnung ist in einem Gehäuse aufgenommen und umfasst eine Testelementaufnahmeeinheit, in der das Testelement zumindest eine Probenaufgabeposition und eine Analyseposition einnehmen kann. Die Testelementaufnahmeeinheit kann z.B. einen in dem Gehäuse enthaltenen Hohlraum definieren, in dem das Testelement in verschiedene Positionen verschoben und dort z.B. zur Probenaufgabe oder Analyse gehalten werden kann. Die Probenaufgabeposition ist diejenige Position des Testelements in der Testelementaufnahmeeinheit, in der eine zu analysierende Probenflüssigkeit auf einen Probenaufnahmeort auf dem Testelement gegeben werden kann. Dabei ragt das Testelement beispielsweise mit einem, den Probenaufnahmeort aufweisenden Ende aus dem Gehäuse des Analysegeräts. In der Analyseposition wird die Probenflüssigkeit auf dem Testelement durch die Messanordnung analysiert. In der Analyseposition kann das Testelement vollständig in das Gehäuse zurückgezogen sein. Die Analyseposition kann aber auch der Probenaufgabeposition entsprechen.

Das Gehäuse umgibt das Analysegerät. Es hat vorzugsweise die Form von handelsüblichen Analysegerätgehäusen und ist kompakt und in einer für den Benutzer leicht zu handhabenden Form ausgebildet. Es enthält die Messanordnung und ggf. ein Vorratsbehältnis zur Aufnahme von unverbrauchten Testelementen. Ferner kann es bei einem integrierten Analysegerät z.B. auch eine Stechhilfe und eine Anzahl von Lanzetten enthalten.

Erfindungsgemäß umfasst das Analysegerät ein beweglich und ggf. lösbar mit dem Gehäuse verbundenes Abfallbehältnis zum Aufnehmen von Testelementen nach der Durchführung von Messungen an der Probenflüssigkeit auf den Testelementen. Die bewegliche oder lösbare Verbindung des Abfallbehältnisses mit dem Gehäuse hat den Vorteil, dass es zur Probenaufgabe auf das Testelement, Entleerung und Reinigung oder zur vollständigen Entsorgung von dem Gehäuse wegbewegt (z.B. weggeklappt) oder gelöst werden kann. Ferner kann das Abfallbehältnis nur im Bedarfsfall (z.B. wenn der Anwender seinen privaten Bereich verlässt, insbesondere auf Reisen, in Schule und Beruf und zu Freizeitaktivitäten) mit dem Analysegerät verbunden werden und das Analysegerät ansonsten ohne das Abfallbehältnis verwendet werden. Durch das Abfallbehältnis werden die benutzten Testelemente automatisch entsorgt bzw. in dem Abfallbehältnis gelagert. Das Abfallbehältnis erweitert z.B. ein handelsübliches Analysegerät. Es ist z.B. eine an das Analysegerät anzubringende Hohlform (z.B. in Form einer Wanne), die die von dem Gerät ausgestoßenen, gebrauchten Testelemente auffängt und verwahrt. Der Anwender braucht sich daher nicht sofort um die Entsorgung von mit Probenflüssigkeit (z.B. Blut) kontaminierten Testelementen zu kümmern. Die Testelemente werden für Dritte unsichtbar aufbewahrt. Im Idealfall bildet das mit dem Gehäuse verbundene Abfallbehältnis eine optische Einheit mit dem Gehäuse des Analysegeräts. Ein passendes äußeres Design des Abfallbehältnisses beeinflusst nicht die Funktionalität des Analysegeräts, erhöht aber die Akzeptanz des Anwenders bzw."tarnt" die Funktion des Analysegeräts.

Das Abfallbehältnis ist bei der vorliegenden Erfindung so angeordnet ist, dass der Probenaufnahmeort eines in der Probenaufgabeposition positionierten Testelements für einen Benutzer zugänglich ist. Somit kann der Benutzer die Probenflüssigkeit problemlos auf den Probenaufnahmeort des in der Probenaufgabeposition befindlichen Testelements auftragen. Dazu kann das Abfallbehältnis während der Probenaufgabe z.B. vom Gehäuse weggeklappt und während der Analyse durch die Messanordnung wieder an das Gehäuse angelegt werden oder eine Öffnung aufweisen, durch die das Testelement zur Probenaufgabe erreichbar ist.

Gemäß einer bevorzugten Ausführungsform der vorliegenden Erfindung sind zur Befestigung des Abfallbehältnisses an dem Gehäuse eine Schnappverbindung, eine Reibkraftverbindung, eine Klettverbindung, Magneten, Klammern, Stecker, Schrauben, Klebeband, Steck- oder Klemmleisten, Bänder oder Schnüren oder Kombinationen daraus vorgesehen. Diese können an dem Gehäuse und/oder an dem Abfallbehältnis vorgesehen sein.

Gemäß einer weiteren Ausführungsform ist das Abfallbehältnis an dem Gehäuse schwenkbar befestigt.

Vorzugsweise ist das Abfallbehältnis ein zumindest einseitig offener Aufsatz, der auf das Gehäuse aufsetzbar und mit diesem beweglich oder lösbar zu verbinden ist.

Das erfindungsgemäße Analysegerät kann ferner eine Transporteinrichtung zum Transport eines Testelements aus dem Vorratsbehältnis in eine Probenaufgabeposition und in eine Analyseposition und zum Ausstoß des Testelements aus dem Gehäuse in das Abfallbehältnis umfassen. Die Transporteinrichtung kann z.B. einen-Stößel oder Schieber enthalten durch den jeweils ein Testelement aus dem Vorratsbehältnis herausgeschoben wird, wobei die anderen in dem Vorratsbehältnis vorhandenen Testelemente weiterhin versiegelt und geschützt in dem Vorratsbehältnis verbleiben. Entsprechende Transporteinrichtungen sind im Stand der Technik umfangreich beschrieben und dem Fachmann in einer Vielzahl von Ausführungsformen geläufig. Die Transporteinrichtung positioniert das entnommene Testelement anschließend in einer Probenaufgabeposition, in der eine Probenflüssigkeit (z.B. Blut oder interstitielle Flüssigkeit) auf einen Probenaufnahmeort auf dem Testelement gegeben wird. In der Probenaufgabeposition ragt das Testelement, z.B. mit einem den Probenaufnahmeort aufweisenden Ende durch einen Schlitz aus dem Gehäuse des erfindungsgemäßen Analysegeräts, damit.der Benutzer die Probe z.B. von seiner Fingerbeere auf den Probenaufnahmeort übertragen kann. Die Transporteinrichtung transportiert das Testelement mit der Probenflüssigkeit danach in eine Analyseposition, in der die Analyse (z.B. elektrochemisch oder photometrisch) der Probe durchgeführt wird. In der Analyseposition kann das Testelement z.B. ganz in das Gehäuse zurückgezogen sein. Die Analyseposition kann aber auch der Probenaufgabeposition entsprechen.

Die Transporteinrichtung dient ferner zum Ausstoß des Testelements aus dem Gehäuse in das Abfallbehältnis. Dazu kann die Transporteinrichtung z.B. einen Federmechanismus aufweisen. Neben einem Federmechanismus kann das Ausstoßen des Testelements auch durch ein einfaches Ausschieben z.B. durch eine Stößel oder Schieber erfolgen, bis es aus der Testelementaufnahmeeinheit in das Abfallbehältnis fällt.

Gemäß einer bevorzugten Ausführungsform der vorliegenden Erfindung umfasst das erfindungsgemäße Analysegerät einen Schlitz in dem Gehäuse, durch den das Testelement aus dem Gehäuse in das Abfallbehältnis ausstoßbar ist. Vorzugsweise ist der Schlitz so ausgebildet, dass das Testelement zur Probenaufnahme teilweise aus dem Schlitz herausragt.

Eine bevorzugte Ausführungsform der vorliegenden Erfindung ist so gestaltet, dass das Abfallbehältnis eine Öffnung enthält, durch die die Probenflüssigkeit auf ein teilweise aus dem Gehäuse ragendes Testelement aufgebracht werden kann. Die Öffnung ist z.B. so ausgebildet, dass der Anwender einen Finger mit einem Tropfen der Probenflüssigkeit durch die Öffnung in das Innere des Abfallbehältnisses stecken kann, um die Probenflüssigkeit auf den Probenaufnahmeort des teilweise aus dem Gehäuse in das Innere des Abfallbehältnisses ragende Testelement zu übertragen. Die bereits verbrauchten Testelemente werden bei dieser Ausführungsform der vorliegenden Erfindung vorzugsweise in einem anderen, von dem teilweise aus dem Gehäuse ragenden, aktuell verwendeten Testelement ausreichend entfernten Bereich des Abfallbehältnisses aufbewahrt. Das Aufbringen der Probenflüssigkeit auf das Testelement durch die Öffnung in dem Abfallbehältnis hat den Vorteil, dass die Analyse einer Probenflüssigkeit noch besser getarnt und im Inneren des Analysegeräts für Dritte unsichtbar durchgeführt werden kann. Der Anwender kann auch bei mit dem Gehäuse verbundenem Abfallbehältnis eine Messung durchführen. Er kann das Abfallbehältnis schon im Voraus mit dem Gehäuse des Analysegeräts verbinden, z.B. bevor er aus dem Haus geht.

Vorzugsweise ist das beweglich zum Gehäuse angeordnete Abfallbehältnis in einer offenen Position zugänglich und nimmt in einer geschlossenen Position die nach der Durchführung der Messung aus dem Gehäuse ausgestoßenen Testelemente auf.

Ferner kann das beweglich zum Gehäuse angeordnete Abfallbehältnis während der Durchführung der Messung durch die Messanordnung und während des Auswerfens des Testelementes aus dem Gehäuse an diesem verbleiben.

Die Geometrie des Testelements, die Probenaufgabeposition und die Analyseposition des Testelements, die Anordnung und Form des Abfallbehältnisses und der Öffnung in dem Abfallbehältnis werden an das jeweilige Analysegerät angepasst. Das Abfallbehältnis ist so ausgebildet, dass keine verbrauchten Testelemente aus dem Abfallbehältnis herausfallen können und dass das Abfallbehältnis sich nicht von selbst von dem Gehäuse löst. Vorzugsweise ist das Abfallbehältnis, insbesondere die Öffnung und/oder sein offenes Ende, manuell oder automatisch durch einen Verschluss verschließbar. Der Verschluss kann z.B. ein Deckel zum Schrauben oder Stecken, ein Schiebeverschluss oder eine Abdeckfolie sein. Der Verschluss verhindert beim Abnehmen des Abfallbehältnisses von dem Gehäuse, z.B. zur Entsorgung verbrauchter Testelemente, dass diese aus dem Abfallbehältnis herausfallen. Vorzugsweise wird das Abfallbehältnis automatisch verschlossen. Es kann aber auch ein Verschluss manuell durch den Anwender angebracht werden. Ein automatischer, d.h. selbstschließender Verschluss, kann z.B. ein Klammer- oder Schnappverschluss sein, ähnlich einem einfachen Brillenetui-Verschluss oder eine durch eine Spiral- oder Blattfeder ausgelöste "Falltür". Ein manueller Verschluss kann z.B. ein klassischer Deckel zum Stecken oder Schrauben oder eine (selbstklebende) Folie mit oder ohne Gummizug sein. Eine weitere alternative oder zusätzliche Möglichkeit ist, dass das Abfallbehältnis eine klebende Innenseite aufweist. Die klebende Innenseite verändert ein Herumfallen der verbrauchten Testelemente in dem Abfallbehältnis, da die Testelemente an der klebenden Innenseite fixiert werden.

Die Erfindung bezieht sich ferner auf ein Abfallbehältnis zum Aufnehmen von Testelementen nach der Durchführung von Messungen in einem ein Gehäuse umfassenden Analysegerät an einer Probenflüssigkeit auf den Testelementen, wobei das Abfallbehältnis Mittel zum beweglichen oder lösbaren Verbinden mit dem Gehäuse des Analysegeräts enthält. Die Mittel zum Verbinden dienen dazu, das Abfallbehältnis von außen an dem Gehäuse des Analysegeräts zu befestigen. Die Mittel zum beweglichen oder lösbaren Verbinden können z.B. eine Schnappverbindung, eine Reibkraftverbindung, eine Klettverbindung, Magneten, Klammern, Stecker, Schrauben, Klebeband, Steck- oder Klemmleisten, Bänder oder Schnüren oder Kombinationen daraus sein.

Die Erfindung bezieht sich weiterhin auf ein Verfahren zur Analyse einer Probenflüssigkeit mittels eines Testelements in einem Analysegerät, das ein Gehäuse umfasst, mit den Schritten
- Aufnehmen der Probenflüssigkeit auf ein Testelement,
- Positionieren des Testelements in eine Analyseposition des Analysegeräts,
- Analyse der Probenflüssigkeit durch eine Messanordnung in dem Analysegerät,
- Ausstoßen des Testelements aus dem Gehäuse des Analysegeräts in ein mit dem Gehäuse beweglich verbundenes Abfallbehältnis.

Insbesondere bezieht sich die Erfindung auf ein Verfahren zur Analyse einer Probenflüssigkeit mittels eines Testelements in einem Analysegerät, das ein Gehäuse umfasst, mit den Schritten
- Entnahme eines Testelements aus einem Vorratsbehältnis in dem Analysegerät,
- Transport des Testelements in eine Probenaufgabeposition,
- Aufnehmen der Probenflüssigkeit auf das Testelement in der Probenaufgabeposition,
- Analyse der Probenflüssigkeit durch eine Messanordnung in dem Analysegerät,
- Ausstoßen des Testelements aus dem Gehäuse des Analysegeräts in ein mit dem Gehäuse beweglich oder lösbar verbundenes Abfallbehältnis.

Anhand der Zeichnung wird die Erfindung nachstehend näher erläutert.

Es zeigen:
- Figuren 1(a) - (e): schematisch ein Analysegerät aus dem Stand der Technik und seine Verwendung,
- Figuren 2(a) und (b): schematisch eine Ausführungsform eines erfindungsgemäßen Analysegeräts,
- Figuren 3(a) und (b): schematisch ein mit dem Gehäuse eines erfindungsgemäßen Analysegeräts verbundenes Abfallbehältnis im Schnitt und
- Figuren 4(a) - (i): verschiedene Möglichkeiten, wie bei einem erfindungsgemäßen Analysegerät ein Abfallbehältnis mit dem Gehäuse beweglich oder lösbar verbunden werden kann.

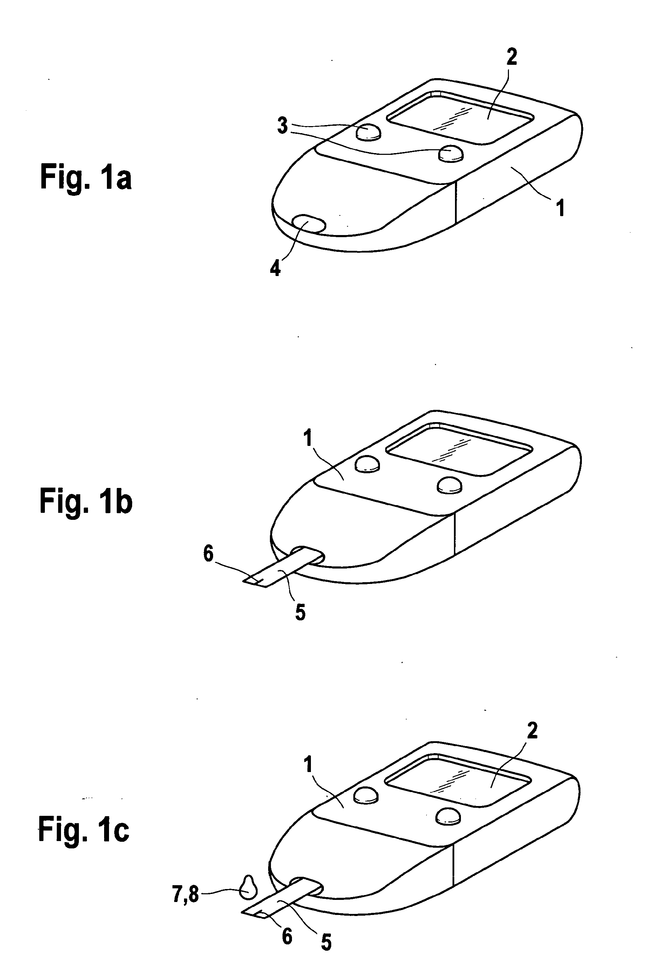

- Figuren 1 (a) - (c): zeigen ein Analysegerät aus dem Stand der Technik.

Das Analysegerät umfasst ein Gehäuse 1, in dem unter anderen eine (nicht dargestellte) zur Analyse einer Probenflüssigkeit auf einem Testelement geeignete Messanordnung angeordnet ist. Figur 1(a) zeigt schematisch den Aufbau des Analysegeräts. Das Gehäuse 1 umfasst eine Anzeige 2 zum Anzeigen von Analyseergebnissen oder sonstigen Informationen für den Anwender. Ferner weist es Knöpfe 3 zur Betätigung des Analysegeräts auf (z.B. zum Starten des Geräts oder zur Menüsteuerung).

In dem Gehäuse 1 ist ein Schlitz 4 ausgebildet, aus dem in Figur 1(b) ein streifenförmiges Testelement 5 teilweise herausragt. Das Testelement 5 befindet sich in einer Probenaufgabeposition, bei der ein Probenaufnahmeort 6 auf dem Testelement 5 für einen Benutzer frei zugänglich ist.

In Figur 1(c) ist die Probenaufgabe auf das Testelement 5 dargestellt. Ein Probenflüssigkeitstropfen 7 (z.B. ein Tropfen Blut, Kontrollflüssigkeit oder interstitielle Flüssigkeit) wird auf den Probenaufnahmeort 6 gegeben. Anschließend findet in dem Analysegerät die Analyse der Probenflüssigkeit 8 statt. Die Analyse kann z.B. elektrochemisch oder photometrisch durchgeführt werden. Das Ergebnis der Analyse wird auf der Anzeige 2 angezeigt.

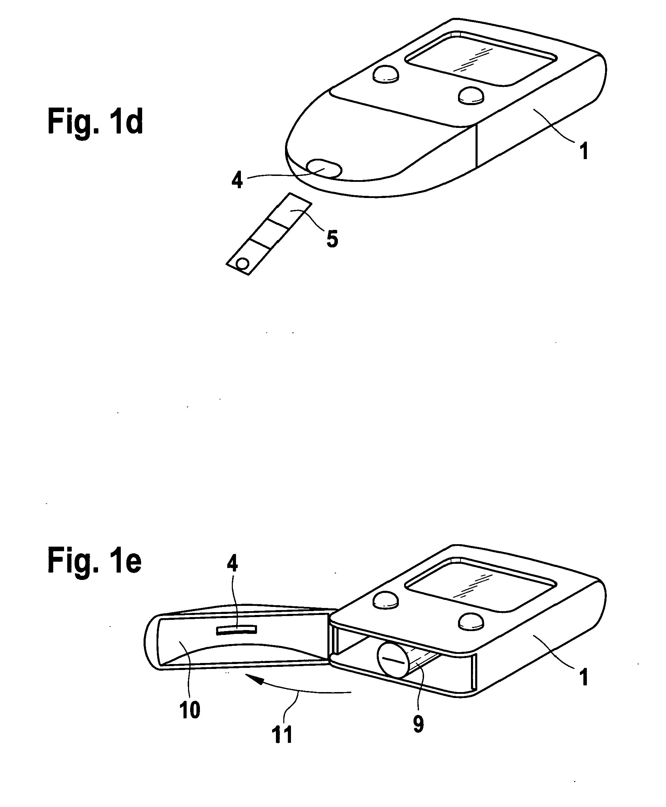

Nach der Messung wird das verbrauchte Testelement 5 aus dem Analysegerät gestoßen (Fig. 1(d)). Der Anwender muss das mit Probenflüssigkeit 8 kontaminierte Testelement 5 entsorgen.

Unverbrauchte Testelemente 5 können in einem Vorratsbehältnis 9 in dem Analysegerät bereitgestellt werden (Fig. 1(e)). Zum Wechseln des Vorratsbehältnisses 9 und/oder Nachfüllen von unverbrauchten Testelementen 5 kann ein Deckel 10 des Gehäuses 1 durch Schwenken in Öffnungsrichtung 11 geöffnet werden. Es können so einzelne Testelemente 5 in das vorhandene Vorratsbehältnis 9 oder ein mit einer Vielzahl von Testelementen 5 gefülltes neues Vorratsbehältnis 9 in das Analysegerät eingelegt werden. Das Analysegerät entnimmt und transportiert die Testelemente 5 automatisch aus dem Vorratsbehältnis 9 mittels einer (nicht dargestellten) in dem Gehäuse 1 vorhandenen Transporteinrichtung. Die Transporteinrichtung transportiert insbesondere jeweils ein Testelement 5 aus dem Vorratsbehältnis 9 in die Probenaufgabeposition (Fig. 1(b)) und nach der Probenaufgabe (Fig. 1(c)) in eine Analyseposition und stößt das verbrauchte Testelement 5 anschließend aus dem Gehäuse 1 aus (Fig. 1(d)). Ein Analysegerät aus dem Stand der Technik gemäß Figur 1 ist z.B. das handelsübliche integrierte System Accu-Chek® Compact von Roche Diagnostics, Deutschland.

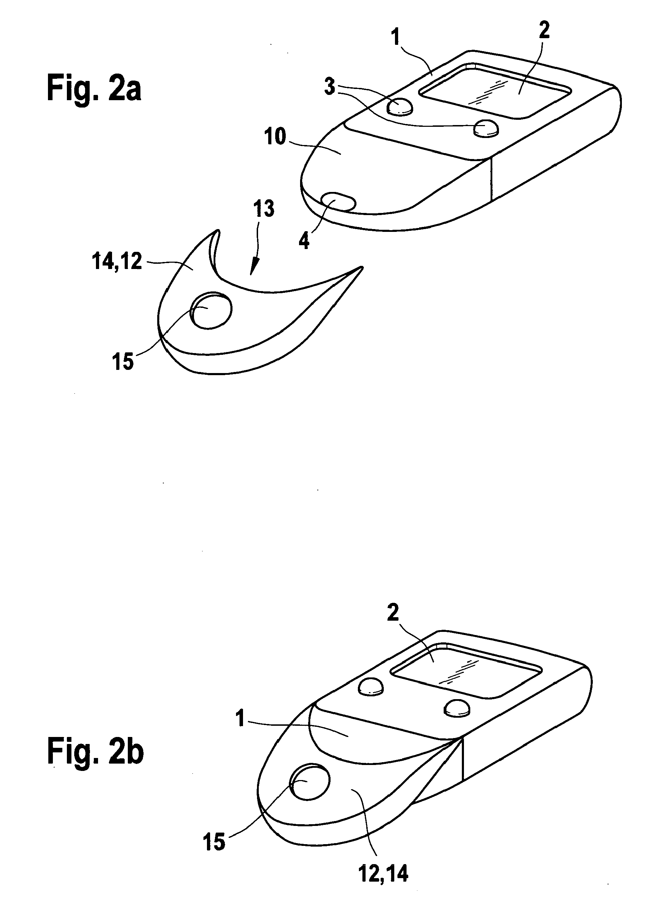

Figuren 2(a) und (b) zeigen eine Ausführungsform eines erfindungsgemäßen. Analysegeräts mit einem Abfallbehältnis.

Die besondere Ausführungsform des erfindungsgemäßen Analysegeräts gemäß Figur 2 entspricht im Wesentlichen dem Analysegerät aus Fig. 1, so dass die Beschreibung zu Fig. 1 ebenfalls für das Analysegerät aus Fig. 2 zutrifft. Das erfindungsgemäße Analysegerät gemäß Figur 2 umfasst zusätzlich ein beweglich und/oder lösbar mit dem Gehäuse 1 verbundenes Abfallbehältnis 12.

In Figur 2(a) sind das Gehäuse 1 und das Abfallbehältnis 12 getrennt und in Fig. 2(b) sind die beiden Komponenten 1, 12 miteinander verbunden dargestellt. Das Abfallbehältnis 12 ist wannenförmig. Es ist ein einseitig offener Aufsatz 14 (offene Seite 13), der auf das Gehäuse 1 aufsetzbar und mit dieser beweglich oder lösbar verbunden ist. Das Gehäuse 1 enthält einen Schlitz 4, durch den ein verbrauchtes Testelement bei aufgesetztem Abfallbehältnis 12 aus dem Gehäuse 1 in das Abfallbehältnis 12 ausstoßbar ist. Das Abfallbehältnis 12 weist eine Öffnung 15 auf, durch die eine Probenflüssigkeit auf einen teilweise aus dem Schlitz 4 in dem Gehäuse 1 ragendes (nicht dargestelltes) Testelement aufgebracht werden kann.

Die Analyse einer Probenflüssigkeit mit einem erfindungsgemäßen Analysegerät gemäß Fig. 2(b) läuft wie folgt ab:
- Entnahme eines Testelements 5 aus einem Vorratsbehältnis 9 in dem Analysegerät,
- Transport des Testelements 5 in eine Probenaufgabeposition,
- Aufnehmen der Probenflüssigkeit auf das Testelement in der Probenaufgabeposition,
- Analyse der Probenflüssigkeit 8 durch eine Messanordnung in dem Analysegerät,
- Ausstoßen des Testelements 5 aus dem Gehäuse 1 des Analysegeräts in das mit dem Gehäuse 1 beweglich oder lösbar verbundene Abfallbehältnis 12.

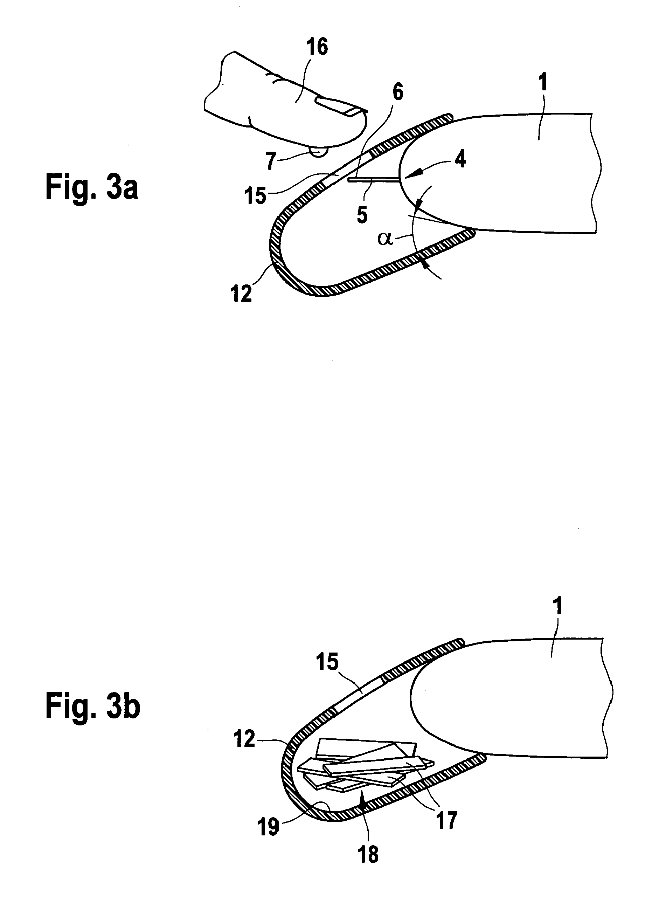

Figuren 3(a) und (b) zeigen ein mit einem Gehäuse eines erfindungsgemäßen Analysegeräts verbundenes Abfallbehältnis im Schnitt. Das Abfallbehältnis 12 ist dabei mit einem Ende des (nur teilweise dargestellten) Gehäuses 1 verbunden. Aus dem Gehäuse 1 ragt in Figur 3(a) ein in Probenaufgabeposition angeordnetes Testelement 5, auf dessen Probenaufnahmeort 6 von dem Finger 16 eines Anwenders ein Probenflüssigkeitstropfen 7 übertragen wird. Das Abfallbehältnis 12 ist um einen Winkel α geneigt an dem Gehäuse 1 beweglich oder lösbar befestigt, so dass der Anwender den Probenaufnahmeort 6 durch die Öffnung 15 problemlos erreichen kann und verbrauchte Testelemente 17 bei einer entsprechenden Ausrichtung des Analysegeräts aufgrund der Schwerkraft in einen von dem Probenaufnahmeort 6 entfemten Saminelbereich 18 gelangen. Die Innenseite 19 des Abfallbehältnisses 12 kann klebend ausgeführt sein, insbesondere im Sammelbereich 18, so dass einmal dort hingelangte verbrauchte Testelemente 17 dort fixiert werden.

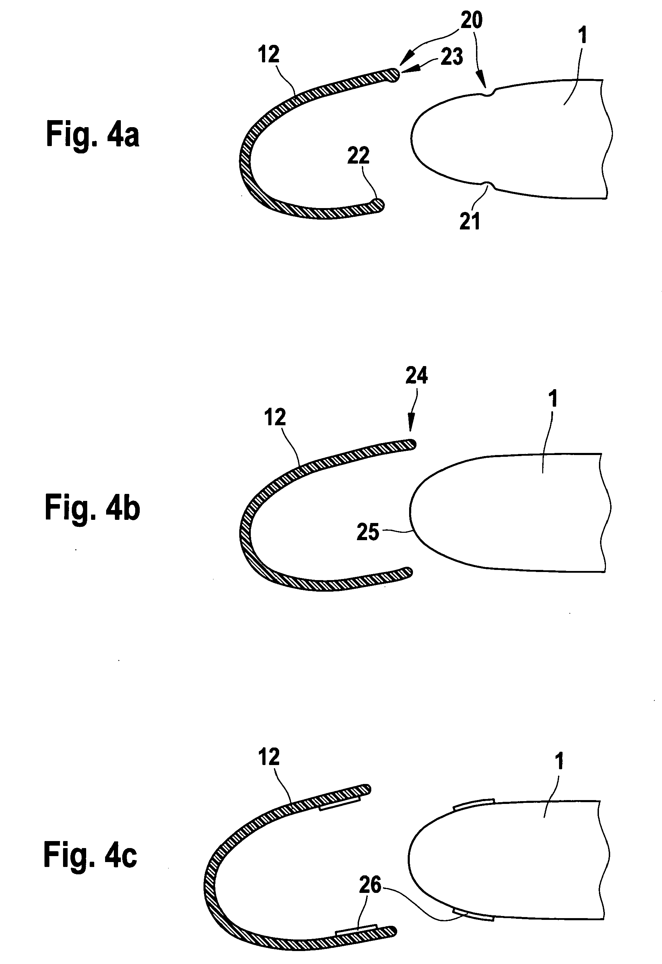

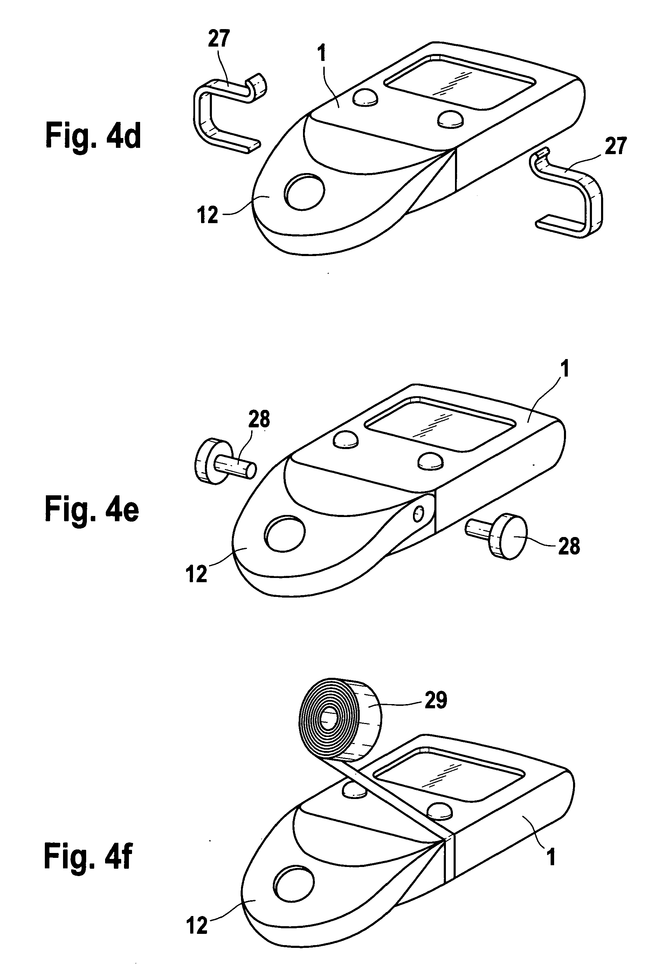

Figuren 4(a) - (i) zeigen verschiedene Möglichkeiten, wie bei einem erfindungsgemäßen Analysegerät ein Abfallbehältnis mit dem Gehäuse beweglich oder lösbar verbunden werden kann. Diese Verbindung verhindert ein unbeabsichtigtes Lösen des Abfallbehältnisses von dem Gehäuse.

In Figur 4(a) ist eine Schnappverbindung 20 dargestellt. Dabei weist das Gehäuse 1 eine Nut 21 auf, in die der mit Wölbungen 22 versehene Rand 23 des Abfallbehältnisses 12 einschnappt.

In Figur 4(b) ist eine Reibkraftverbindung 24 dargestellt. Dabei wird das Abfallbehältnis 12 auf das Ende 25 des Gehäuses 1 geschoben und dort durch die Reibung gehalten. Dazu kann das Ende 25 z.B. auf seiner Oberfläche aufgeraut oder mit einem entsprechenden "rutschfesten" Material (z.B. Kunststoff oder Gummi) bedeckt sein.

Figur 4(c) zeigt eine Klettverbindung oder Verbindung mittels Magneten. Dabei weist sowohl das Abfallbehältnis 12 als auch das Gehäuse 1 Klettelemente oder Magneten 26 auf.

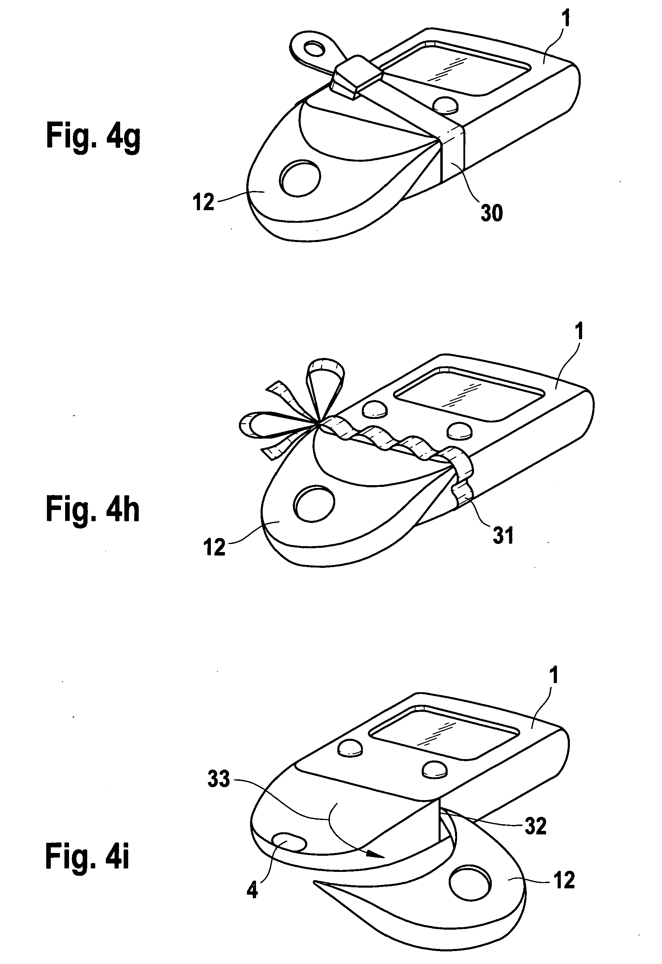

Weitere für die vorliegende Erfindung verwendbare Mittel zum beweglichen oder lösbaren Verbinden des Abfallbehältnisses mit dem Analysegerät sind
- Klammern 27 aus Metall oder Kunststoff (Fig. 4(d)),
- Stecker oder Schrauben 28 (Fig. 4(e)),
- Klebeband 29 (Fig. 4(f)),
- alle Arten von Steck- oder Klemmleisten 30 (z.B. in Form eines Reißverschlusses) (Fig. 4(g)) und
- alle Arten von Bändern oder Schnüren 31 (Fig. 4(h)).

Das mit gebrauchten Testelementen gefüllte Abfallbehältnis 12 wird zur Entsorgung der gebrauchten Testelemente von dem Gehäuse 1 abgenommen. Das Abfallbehältnis 12 kann entweder als Ganzes entsorgt und durch ein neues Abfallbehältnis 12 ersetzt werden oder es kann entleert, gereinigt und wieder verwendet werden.

Fig. 4(i) zeigt ein Abfallbehältnis 12, das an dem Gehäuse 1 schwenkbar in Schwenkrichtung 33 um eine Achse 32 befestigt ist. Das ist z.B. vorteilhart, um bei Problemen mit einem Testelement (Verklemmen in dem Schlitz 4 oder ähnlichem), dieses besser zu erreichen.

### Bezugszeichenliste

- 1: Gehäuse
- 2: Anzeige
- 3: Knöpfe
- 4: Schlitz
- 5: Testelement
- 6: Probenaufnahmeort
- 7: Probenflüssigkeitstropfen
- 8: Probenflüssigkeit
- 9: Vorratsbehältnis
- 10: Deckel
- 11: Öffnungsrichtung
- 12: Abfallbehältnis
- 13: offene Seite
- 14: Aufsatz
- 15: Öffnung
- 16: Finger
- 17: verbrauchte Testelemente
- 18: Sammelbereich
- 19: Innenseite
- 20: Schnappverbindung
- 21: Nut
- 22: Wölbungen
- 23: Rand
- 24: Reibkraftverbindung
- 25: Ende des Gehäuses
- 26: Klettelemente oder Magneten
- 27: Klammern
- 28: Stecker oder Schrauben
- 29: Klebeband
- 30: Steck- und Klemmleisten
- 31: Bänder oder Schnüren
- 32: Achse
- 33: Schwenkrichtung

## Patentansprüche

1. Analysegerät zur Analyse einer Probenflüssigkeit (8) mittels eines Testelements (5), umfassend eine Messanordnung zur Durchführung von Messungen an einer auf einem Testelement (5) aufgenommenen Probenflüssigkeit (8), wobei die Messanordnung in ein Gehäuse (1) aufgenommen ist und eine Testelementaufnahmeeinheit umfasst, in der ein Testelement zumindest eine Probenaufgabeposition und eine Analyseposition einnehmen kann, **gekennzeichnet durch** ein beweglich mit dem Gehäuse (1) verbundenes Abfallbehältnis (12) zum Aufnehmen von Testelementen (5, 17) nach der Durchführung von Messungen an der Probenflüssigkeit (8) auf einem Testelement (5) in der Analyseposition, wobei das Abfallbehältnis (12) so angeordnet ist, dass ein Probenaufnahmeort eines in der Probenaufgabeposition positionierten Testelements (5) zugänglich ist.

2. Analysegerät gemäß Anspruch 1, **dadurch gekennzeichnet, dass** das Abfallbehältnis (12) lösbar mit dem Gehäuse (1) verbunden ist.

3. Analysegerät gemäß einem der Ansprüche 1 oder 2, **gekennzeichnet durch** ein Vorratsbehältnis (9) zur Aufnahme von für die Analyse bereitgestellten Testelementen (5).

4. Analysegerät gemäß einem der Ansprüche 1 bis 3, **dadurch gekennzeichnet, dass** zur Befestigung des Abfallbehältnisses (12) an dem Gehäuse (1) eine Schnappverbindung (20), eine Reibkraftverbindung (24), eine Klettverbindung, Magneten (26), Klammern (27), Stecker, Schrauben (28), Klebeband (29), Steck- oder Klemmleisten (30), Bänder oder Schnüren (31) oder Kombinationen daraus vorgesehen sind.

5. Analysegerät gemäß einem der Ansprüche 1 bis 4, **dadurch gekennzeichnet, dass** das Abfallbehältnis (12) an dem Gehäuse (1) schwenkbar befestigt ist.

6. Analysegerät gemäß einem der Ansprüche 1 bis 5, **dadurch gekennzeichnet, dass** das Abfallbehältnis (12) ein zumindest einseitig offener Aufsatz (14) ist, der auf das Gehäuse (1) aufsetzbar und mit diesem lösbar zu verbinden ist.

7. Analysegerät gemäß einem der Ansprüche 3 bis 6, **gekennzeichnet durch** eine Transporteinrichtung zum Transport eines Testelements (5) in die Analyseposition und zum Ausstoß des Testelements (5) aus dem Gehäuse (1) in das Abfallbehältnis (12).

8. Analysegerät gemäß einem der Ansprüche 1 bis 7, **gekennzeichnet durch** einen-Schlitz (4) in dem Gehäuse (1), **durch** den das Testelement (5) aus dem Gehäuse (1) in das Abfallbehältnis (12) ausstoßbar ist.

9. Analysegerät gemäß Anspruch 8, **dadurch gekennzeichnet, dass** der Schlitz (4) so ausgebildet ist, dass das Testelement (5) zur Probenaufnahme teilweise aus dem Schlitz (4) herausragt.

10. Analysegerät gemäß einem der Ansprüche 1 bis 9, **dadurch gekennzeichnet, dass** das beweglich zum Gehäuse (1) angeordnete Abfallbehältnis (12) eine Öffnung (15) enthält, durch die die Probenflüssigkeit (8) auf ein teilweise aus dem Gehäuse (1) ragendes Testelement (5) aufgebracht werden kann wobei das beweglich zum Gehäuse (1) angeordnete Abfallbehältnis (12) während der Messung am Gehäuse (1) verbleibt.

11. Analysegerät gemäß einem der Ansprüche 1 bis 10, **dadurch gekennzeichnet, dass** das beweglich zum Gehäuse (1) angeordnete Abfallbehältnis (12) in einer offenen Position zugänglich ist und in einer geschlossenen Position die nach der Durchführung der Messung aus dem Gehäuse (1) ausgestoßenen Testelemente (5) aufnimmt.

12. Analysegerät gemäß einem der Ansprüche 1 bis 11, **dadurch gekennzeichnet, dass** das beweglich zum Gehäuse (1) angeordnete Abfallbehältnis (12) während der Durchführung der Messung durch die Messanordnung und während des Auswerfens des Testelementes (5) aus dem Gehäuse (1) an diesem verbleibt.

13. Analysegerät gemäß einem der Ansprüche 1 bis 12, **dadurch gekennzeichnet, dass** das Abfallbehältnis (12) manuell oder automatisch durch einen Verschluss verschließbar ist.

14. Analysegerät gemäß Anspruch 13, **dadurch gekennzeichnet, dass** der Verschluss ein Deckel zum Schrauben oder Stecken, ein Schiebeverschluss oder eine Abdeckfolie ist.

15. Analysegerät gemäß einem der Ansprüche 1 bis 14, **dadurch gekennzeichnet, dass** das Abfallbehältnis (12) eine klebende Innenseite (19) aufweist.

16. Abfallbehältnis (12) zum Aufnehmen von Testelementen (5, 17) nach der Durchführung von Messungen in einem ein Gehäuse (1) umfassenden Analysegerät an einer Probenflüssigkeit (8) auf den Testelementen (5), **dadurch gekennzeichnet, dass** das Abfallbehältnis (12) Mittel zum beweglichen Verbinden mit dem Gehäuse (1) des Analysegeräts enthält.

17. Verfahren zur Analyse einer Probenflüssigkeit (8) mittels eines Testelements (5) in einem Analysegerät, das ein Gehäuse (1) umfasst, **gekennzeichnet durch**
- Aufnehmen der Probenflüssigkeit (8) auf ein Testelement (5),
- Positionieren des Testelements (5) in eine Analyseposition des Analysegeräts,
- Analyse der Probenflüssigkeit (8) **durch** eine Messanordnung in dem Analysegerät,
- Ausstoßen des Testelements (5) aus dem Gehäuse (1) des Analysegeräts in ein mit dem Gehäuse (1) beweglich verbundenes Abfallbehältnis (12).
